(19) Europäisches Patentamt
European Patent Office
Office européen des brevets

(11) Veröffentlichungsnummer: **0 422 492 A1**

# (12) EUROPÄISCHE PATENTANMELDUNG

(21) Anmeldenummer: **90118943.1**

(22) Anmeldetag: **04.10.90**

(51) Int. Cl.5: **C07D 401/04**, C07D 211/88, C08K 5/3435

(30) Priorität: **13.10.89 DE 3934176**

(43) Veröffentlichungstag der Anmeldung: **17.04.91 Patentblatt 91/16**

(84) Benannte Vertragsstaaten: **BE CH DE FR GB IT LI NL**

(71) Anmelder: **BASF Aktiengesellschaft**
**Carl-Bosch-Strasse 38**
**W-6700 Ludwigshafen(DE)**

(72) Erfinder: **Aumueller, Alexander, Dr.**
**An der Marlach 5**
**W-6705 Deidesheim(DE)**
Erfinder: **Trauth, Hubert**
**Milanstrasse 6**
**W-6724 Dudenhofen(DE)**

## (54) Cyclische Amide.

(57) Cyclische Amide I

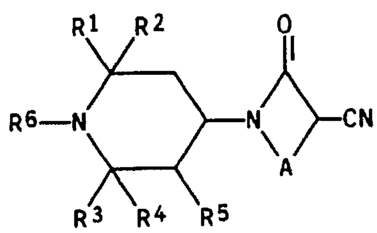

I

in der die Variablen folgende Bedeutung haben:
A eine Gruppierung der folgenden Formel

EP 0 422 492 A1

(a) 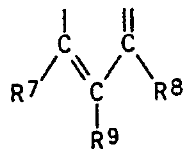

(b) 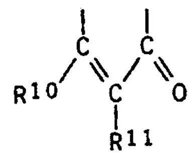

(c) 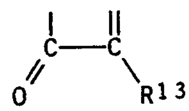

(d) 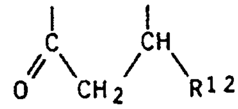

(e) 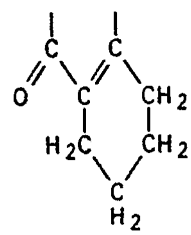

wobei jeweils die linke freie Bindung die Verknüpfung mit dem Amid-Stickstoff herstellt, die rechte freie Bindung je nach Gruppierung eine Einfach- oder eine Doppelbindung ist und wobei die Reste $R^7$ bis $R^{12}$ für Wasserstoff, $C_1$-$C_4$-Alkylgruppen, Phenylgruppen und der Rest $R^{13}$ für einen der Reste $R^7$ bis $R^{12}$ oder eine Hydroxylgruppe steht

$R^1$ bis $R^4$ $C_1$-$C_4$-Alkylgruppen, wobei $R^1$ und $R^2$ und/oder $R^3$ und $R^4$ jeweils zu einem fünf- oder sechsgliedrigen Ring verbunden sein können

$R^5$ Wasserstoff oder eine $C_1$-$C_3$-Alkylgruppe

$R^6$ Wasserstoff, eine $C_1$-$C_4$-Alkylgruppe, die zusätzlich durch eine Cyano-, Hydroxyl- oder Aminogruppe oder einen Phenylrest substituiert sein kann, oder eine $C_1$-$C_4$-Acylgruppe.

Die cyclischen Amide I dienen als Stabilisatoren für organische Materialien.

# CYCLISCHE AMIDE

Die vorliegende Erfindung betrifft neue cyclische Amide der allgemeinen Formel I

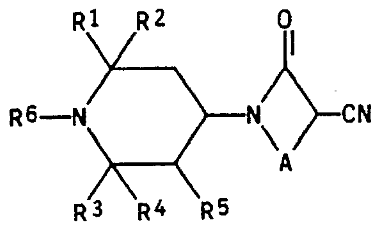

I

in der die Variablen folgende Bedeutung haben:
A eine Gruppierung der folgenden Formel

(a)

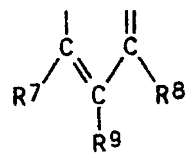

(b)

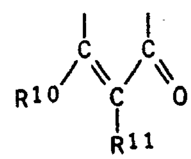

(c)

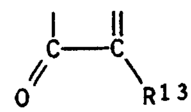

(d)

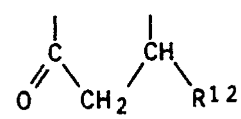

(e)

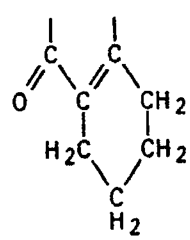

wobei jeweils die linke freie Bindung die Verknüpfung mit dem Amid-Stickstoff herstellt, die rechte freie Bindung je nach Gruppierung eine Einfach- oder eine Doppelbindung ist und wobei die Reste $R^7$ bis $R^{12}$ für Wasserstoff, $C_1$-$C_4$-Alkylgruppen, Phenylgruppen und der Rest $R^{13}$ für einen der Reste $R^7$ bis $R^{12}$ oder eine Hydroxylgruppe steht

$R^1$ bis $R^4$ $C_1$-$C_4$-Alkylgruppen, wobei $R^1$ und $R^2$ und/oder $R^3$ und $R^4$ jeweils zu einem fünf- oder sechsgliedrigen Ring verbunden sein können

$R^5$ Wasserstoff oder eine $C_1$-$C_3$-Alkylgruppe

$R^6$ Wasserstoff, eine $C_1$-$C_4$-Alkylgruppe, die zusätzlich durch eine Cyano-, Hydroxyl- oder Aminogruppe oder einen Phenylrest substituiert sein kann, oder eine $C_1$-$C_4$-Acylgruppe.

Weiterhin betrifft die Erfindung ein Verfahren zur Herstellung dieser Verbindungen, ihre Verwendung als

Stabilisatoren für organische Materialien sowie gegen die Einwirkung von Licht und Wärme stabilisierte organische Materialien, die diese Verbindungen enthalten.

Es ist bekannt, daß Polyalkylpiperidin-Derivate Kunststoffe vor Zerstörung durch Licht und Wärme schützen, z.B. aus den älteren deutschen Patentanmeldungen P 38 23 112, P 38 28 536, P 38 42 304 und P 38 44 355 sowie der DE-A 38 01 944 und der DE-B 19 29 928.

Unbefriedigend ist oft die Verträglichkeit dieser Piperidin-Derivate mit bestimmten Kunststoffarten, beispielsweise mit Polyolefinen oder Polyamiden, die Dauer der Schutzwirkung, die Eigenfarbe der Substanzen sowie die Neigung zur Flüchtigkeit und die thermische Zersetzung solcher Stabilisatoren beim Einarbeiten in Kunststoffe bei erhöhter Temperatur.

Der Erfindung lag daher die Aufgabe zugrunde, neue Stabilisatoren bereitzustellen, welche die genannten Nachteile nicht aufweisen.

Demgemäß wurden die eingangs beschriebenen neuen cyclischen Amide I gefunden.

Die Reste $R^1$ bis $R^4$ stehen für lineare oder verzweigte $C_1$-$C_4$-Alkylgruppen wie Methyl, Ethyl, n-Propyl, iso-Propyl, n-Butyl oder iso-Butyl. Weiterhin können $R^1$ und $R^2$ und/oder $R^3$ und $R^4$ jeweils zu einem fünf- oder sechsgliedrigem Ring, vorzugsweise einem Cyclopentan- oder Cyclohexanring, verbunden sein.

$R^5$ bezeichnet Wasserstoff, Methyl, Ethyl, n-Propyl oder iso-Propyl.

$R^6$ bedeutet Wasserstoff, eine wie oben bezeichnete $C_1$-$C_4$-Alkylgruppe, eine substituierte $C_1$-$C_4$-Alkylgruppe, beispielsweise Cyanomethyl, $\beta$-Cyanoethyl, Hydroxymethyl, $\beta$-Hydroxyethyl, $\beta$-Aminoethyl oder Benzyl, oder eine $C_1$-$C_4$-Acylgruppe wie Formyl, Acetyl, Propionyl oder Butyryl.

$R^7$ bis $R^{12}$ stehen jeweils für Wasserstoff, wie oben bezeichnete $C_1$-$C_4$-Alkylgruppen oder Phenylreste.

$R^{13}$ hat die Bedeutung von $R^7$ bis $R^{12}$, zusätzlich kann es auch eine Hydroxylgruppe bezeichnen.

Bevorzugte Verbindungen I sind solche, bei denen die Variablen folgende Bedeutung haben:

$R^1$ bis $R^4$ Methylgruppen
$R^5$ und $R^6$ Wasserstoff
$R^7$, $R^8$ u. $R^{10}$ Methyl- oder Ethylgruppen oder Phenylreste
$R^9$ und $R^{11}$ Wasserstoff oder Methylgruppen
$R^{12}$ eine Phenylgruppe
$R^{13}$ eine Hydroxylgruppe.

Von den Gruppierungen A werden (a) und (b) bevorzugt.

Weist das cyclische Amid I ein acides Proton auf, so kann die Verbindung auch in einer tautomeren Form und/oder in einer Betain-Struktur vorliegen.

Beispielsweise kann die Verbindung Ia als tautomeres inneres Salz der Formel Ib vorliegen.

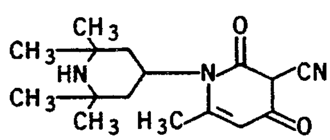
Ia

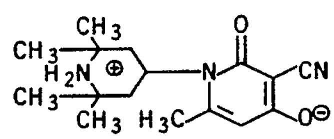
Ib

Die cyclischen Amide I können außer in Form der freien Basen und in Betainform auch als Piperidinium-Salze mit Fremdanionen sowie als Hydrate vorliegen. Geeignete Anionen stammen beispielsweise aus anorganischen Säuren und vorzugsweise aus Carbonsäuren und Sulfonsäuren.

Beispiele für anorganische Anionen sind Chlorid, Bromid, Sulfat, Methansulfonat, Tetrafluorborat, Phosphat und Rhodanid. Als Sulfonatanionen eignen sich z.B. Benzolsulfonat und Tosylat. Als Carboxylatanionen dienen beispielsweise Formiat, Acetat, Propionat, Hexanoat, Cyclohexanoat, Lactat, Stearat, Dodecylbenzoat, Benzoat, Acrylat, Methacrylat, Citrat, Malonat und Succinat sowie Anionen von Polycarbonsäuren mit bis zu etwa 3000 Carboxylgruppen.

Die erfindungsgemäßen cyclischen Amide I stellt man zweckmäßigerweise durch Umsetzung von $\alpha$-Cyano-N-(polyalkylpiperidyl)acetamiden der allgemeinen Formel II

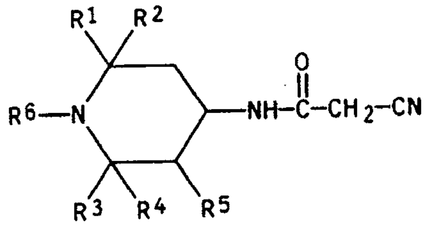

II,

(a) mit einer 1,3-Dicarbonylverbindung, beispielsweise Acetylaceton, Hexan-2,4-dion, Heptan-3,5-dion, Benzoylaceton, Dibenzoylmethan, Malondialdehyd oder Acetylacetaldehyd,

(b) mit einem β-Carbonylcarbonsäureester, beispielsweise dem Methyl- oder Ethylester der Acetessigsäure, 3-Oxopentansäure, 2-Methylacetessigsäure, Benzoylessigsäure oder Formylessigsäure,

(c) mit einem Oxalsäurediester wie Oxalsäuredimethyl- oder -diethylester oder einem α-Carbonylcarbonsäureestern, beispielsweise dem Methyl- oder Ethylester der 2-Oxopropionsäure, Benzoylameisensäure oder Glyoxylsäure,

(d) mit einem olefinisch α,β-ungesättigten Carbonsäureester, beispielsweise dem Methyl- oder Ethylester der Zimtsäure, Acrylsäure oder Crotonsäure, oder

(e) mit einem Cyclohexanon-2-carbonsäureester, beispielsweise dem entsprechenden Methyl- oder Ethylester in an sich bekannter Weise her.

In der Regel verwendet man für die Umsetzung einen basischen Katalysator wie ein Amin, Ammoniak oder ein Alkalimetallhydroxid, -alkoholat oder -carbonat. Als Amine eignen sich beispielsweise Ethylamin, Diethylamin, Triethylamin, Trimethylamin, Morpholin und vor allem Piperidin und Pyridin. Von den basischen Alkalimetallverbindungen eignen sich Natriumhydroxid, Kaliumhydroxid, Natriumcarbonat, Kaliumcarbonat, Natriumethylat, Kalium-tert.-butylat und besonders Natriummethylat.

Üblicherweise führt man die Umsetzung in einem Lösungsmittel wie einem Alkohol, beispielsweise Methanol, Ethanol, n-Propanol, iso-Propanol, n-Butanol oder iso-Butanol, einem aromatischen Kohlenwasserstoff oder Halogenkohlenwasserstoff, beispielseise Benzol, Toluol, Xylol oder Chlorbenzol, in der umzusetzenden und im Überschuß vorliegenden Carbonyl- bzw. Carbonsäureester-Verbindung oder in Wasser durch. Man arbeitet normal erweise bei Temperaturen von 20 bis 180° C, insbesondere 100 bis 160° C.

Die erfindungsgemäßen cyclischen Amide I eignen sich zum Stabilisieren von organischen Materialien, beispielsweise von kosmetischen Präparaten wie Salben, Arzneimittelformulierungen oder Vorprodukten für Kunststoffe und Lacke, insbesondere jedoch von Kunststoffen und Lacken selbst, gegen die Einwirkung von Licht und Wärme. Sie können auch Metallionen in den organischen Materialien desaktivieren.

Als Kunststoffe, die durch die Verbindungen I stabilisiert werden können, seien beispielsweise genannt

- Polymere von Mono- und Diolefinen, beispielsweise Polyethylen niedriger oder hoher Dichte, lineares Polybut-1-en, Polyisopren und Polybutadien, sowie Copolymere von Mono- oder Diolefinen oder Mischungen der genannten Polymeren
- Copolymere von Mono- oder Diolefinen mit Vinylmonomeren, beispielsweise Ethylen-Alkylacrylat-Copolymere, Ethylen-Alkylmethacrylat-Copolymere, Ethylen-Vinylacetat-Copolymere und Ethylen-Acrylsäure-Copolymere
- Polystyrol
- Copolymere von Styrol oder α-Methylstyrol mit Dienen oder Acrylsäurederivaten, beispielsweise Styrol-Butadien-Copolymere, Styrol-Acrylnitril-Copolymere, Styrol-Ethylmethacrylat-Copolymere, Styrol-Butadien-Ethylacrylat-Copolymere und Styrol-Acrylnitril-Methacrylat-Copolymere
- Acrylnitril-Butadien-Styrol-Copolymere, Methylmethacrylat-Butadien-Styrol-Copolymere und ähnliche Polymere
- halogenhaltige Polymere, z.B. Polyvinylchlorid, Polyvinylfluorid, Polyvinylidenchlorid, Polyvinylidenfluorid sowie deren Copolymere
- Polymere, die sich von α,β-ungesättigten Säuren und deren Derivate ableiten, z.B. Polyacrylate, Polymethacrylate, Polyacrylamide und Polyacrylnitrile
- Polymere, die sich von ungesättigten Alkoholen und Aminen ableiten, beispielsweise Polyvinylalkohol und Polyvinylpyrrolidon
- sonstige Polymere von vinylgruppenhaltigen Monomeren, beispielsweise Polyvinylacetat
- Polymere wie Polyurethane, Polyamide, Polyharnstoffe, Polyester, Polycarbonate, Polysulfone, Polyethersulfone und Polyetherketone.

Bevorzugt können die cyclischen Amide I zum Stabilisieren von Polyamiden verwendet werden.

Weiterhin können mit den Verbindungen I Lacküberzüge stabilisiert werden, beispielsweise Industrielak-

kierungen. Unter diesen sind Einbrennlackierungen für den Fahrzeugbau, vorzugsweise Zweischichtlackierungen, besonders hervorzuheben.

Das Vermischen der cyclischen Amide I mit den Kunststoffen und Lacken erfolgt nach den dem Fachmann bekannten Methoden. Dabei können die Verbindungen I in fester oder, speziell bei Lacken, gelöster Form zugesetzt werden. Die gute Löslichkeit der Verbindungen I in Lacksystemen ist hierbei von besonderem Vorteil.

Gegenstand dieser Erfindung sind außerdem gegen die Einwirkung von Licht und Wärme stabilisierte organische Materialien, insbesondere Kunststoffe und Lacke, welche, bezogen auf die Menge des organischen Materials, 0,01 bis 5 Gew.-%, vorzugsweise 0,02 bis 2 Gew.-% eines oder mehrerer cyclischer Amide I enthalten.

Außer den Verbindungen I können die organischen Materialien noch weitere Hilfs- und Füllstoffe in den hierfür üblichen Mengen enthalten, beispielsweise:

- weitere Lichtschutzmittel wie 2-(2′-Hydroxyphenyl)benztriazole, 2,4-Bis(2′-hydroxyphenyl)-6-alkyl-s-triazine, 2-Hydroxybenzophenone oder 1,3-Bis(2′-hydroxybenzoyl)benzole
- Antioxidantien auf der Basis von Phosphor- und Schwefelverbindungen und von sterisch gehinderten Phenolen
- Metalldesaktivatoren wie N,N-Dibenzaloxalsäuredihydrazid oder N,N-Bis[3-(3′,5′-di-tert.-butyl-4′-hydroxyphenyl)propionyl]hydrazin
- Füllstoffe wie Ruß, Kaolin, Talk oder Glasfasern
- Pigmente wie Titandioxid
- Sonstige Zusatzstoffe wie Weichmacher, Gleitmittel, Emulgatoren, optische Aufheller, Flammschutzmittel oder Antistatica.

Als phosphorhaltige Antioxidantien sind z.B.
Tris(nonylphenyl)phosphit,
Distearylpentaerythritdiphosphit,
Tris(2,4-di-tert.-butylphenyl)phosphit,
Tris(2-tert.-butyl-4-methylphenyl)phosphit,
Bis(2,4-di-tert.-butylphenyl)pentaerythritdiphosphit und
Tetrakis(2,4-di-tert.-butylphenyl)-4,4′-biphenylendiphosphit
zu nennen.

Als schwefelhaltige Antioxidationsmittel sind z.B.
Dilaurylthiodipropionat,
Dimyristylthiodipropionat,
Distearylthiodipropionat,
Pentaerythrittetrakis(β-laurylthiopropionat) und
Pentaerythrittetrakis(β-hexylthiopropionat)
zu nennen.

Als sterisch gehinderte phenolische Antioxidationsmittel kommen z.B.
2,6-Di-tert.-butyl-4-methylphenol,
n-Octadecyl-β-(3,5-di-tert.-butyl-4-hydroxyphenyl)propionat,
1,1,3-Tris(2-methyl-4-hydroxy-5-tert.-butylphenyl)butan,
1,3,5-Trimethyl-2,4,6-tris(3,5-di-tert.-butyl-4-hydroxybenzyl)benzol,
1,3,5-Tris(3,5-di-tert.-butyl-4-hydroxybenzyl)isocyanurat,
1,3,5-Tris[(3,5-di-tert.-butyl-4-hydroxyphenyl)propionyloxy-ethyl]isocyanurat,
1,3,5-Tris(2,6-di-methyl-3-hydroxy-4-tert.-butylbenzyl)isocyanurat und
Pentaerythrit-tetrakis[β-(3,5-di-tert.-butyl-4-hydroxyphenyl)propionat]
in Betracht.

Die erfindungsgemäßen cyclischen Amide I zeichnen sich durch eine gute Verträglichkeit mit den üblichen Kunststoffarten und durch eine gute Löslichkeit in den üblichen Lacksystemen aus. Sie haben in der Regel keine oder nur eine sehr geringe Eigenfarbe, sind bei den üblichen Kunst stoff- und Lack-Verarbeitungstemperaturen stabil und nicht flüchtig und bewirken vor allen Dingen eine lange Schutzdauer der mit ihnen behandelten Materialien.

Herstellungsbeispiele

Allgemeine Arbeitsvorschrift für die Beispiele 1 bis 8

0,25 mol α-Cyano-N-(2,2,6,6-tetramethyl-4-piperidyl)acetamid, hergestellt aus Cyanessigsäureethylester und 2,2,6,6-Tetramethyl-4-aminopiperidin durch Erhitzen in ethanolischer Lösung, wurde mit einer der in Tabelle 1 bezeichneten Carbonyl- oder Carbonsäureester-Verbindungen in Gegenwart eines basischen Katalysators, gegebenenfalls in einem Lösungsmittel, bei den angegebenen Reaktionsbedingungen umgesetzt und aufgearbeitet.

Einzelheiten zu diesen Beispielen sowie zu den erhaltenen Produkten sind der Tabelle 1 zu entnehmen. Die Herstellungsbedingungen und die Ausbeuten wurden nicht optimiert.

Tabelle 1

Herstellung von cyclischen Amiden I der Formel

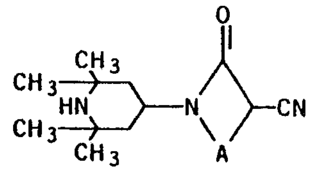

| Bsp.-Nr. | A | Carbonyl- oder Carbonsäure-ester-Verbindung (Menge) | Lösungsmittel (Menge) | basischer Katalysator (Menge) | Reaktionsbedingungen | Aufarbeitung | Ausbeute | Farbe | Schmelzpunkt |
|---|---|---|---|---|---|---|---|---|---|
| 1 | 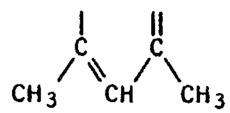 | Acetylaceton (12,5 g) | Wasser (85 ml) | Piperidin (4 ml) | 17,5 h/100°C | A | 10 g | farblos | 180-182°C |
| 2 | 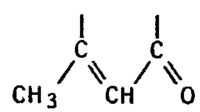 | Acetessigsäuremethylester (160 ml) | - | Piperidin (5 ml) | 1) 6,5 h/100°C<br>2) 3,5 h/150°C | B | 29 g | farblos | > 320°C |
| 3 | 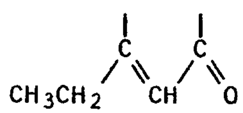 | 3-Oxopentansäuremethylester (100 ml) | - | Pyridin (5 ml) | 6,5 h/145°C | C | 30 g | farblos | > 310°C |

Tabelle 1 (Fortsetzung)

| Bsp.-Nr. | A | Carbonyl- oder Carbonsäure-ester-Verbindung (Menge) | Lösungs-mittel (Menge) | basischer Katalysator (Menge) | Reaktions-bedingungen | Auf-arbei-tung | Aus-beute | Farbe | Schmelz-punkt |
|---|---|---|---|---|---|---|---|---|---|
| 4 | 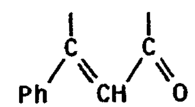 | Benzoylessigsäure-ethylester (100 ml) | - | Pyridin (5 ml). | 12,5 h/160°C | C | 6 g | farb-los | > 320°C |
| 5 | 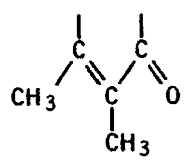 | 2-Methylacetessig-säuremethylester (160 ml) | - | Pyridin (5 ml) | 1) 6 h/100°C<br>2) 5 h/150°C | D | 16 g | farb-los | > 320°C |
| 6 | 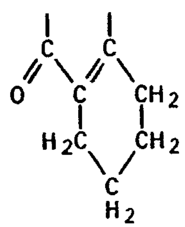 | Cyclohexanon-2-carbonsäureethylester (125 ml) | - | Pyridin (5 ml) | 17,5 h/145°C | E | 15 g | farb-los | > 320°C |

Tabelle 1 (Fortsetzung)

| Bsp.-Nr. | A | Carbonyl- oder Carbonsäure-ester-Verbindung (Menge) | Lösungsmittel (Menge) | basischer Katalysator (Menge) | Reaktionsbedingungen | Aufarbeitung | Ausbeute | Farbe | Schmelzpunkt |
|---|---|---|---|---|---|---|---|---|---|
| 7 | 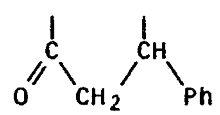 | Zimtsäuremethylester (40,5 g) | Methanol (130 ml) | Natrium-methylat (1,4 g) | 48 h/25°C | F | 14 g | farblos | 146°C |
| 8 | 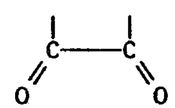 | Oxalsäurediethylester (310 ml) | - | Pyridin (5 ml) | 14,5 h/150°C | G | 36 g | gelblich | > 320°C |

Die einzelnen Reaktionsansätze wurden nach folgenden Methoden aufgearbeitet:

A: nach Abkühlung Abfiltrieren des Niederschlages, Waschen mit Wasser und Umkristallisieren aus Acetonitril

B: nach Abkühlung Abfiltrieren des Niederschlages, Waschen mit Methanol und Aceton, Umkristallisieren aus Essigsäure/Ameisensäure (1,7:1), Lösen des umkristallisierten Produktes in 15 gew.-%iger Natronlauge und Wiederausfällen mit Essigsäure bei pH 10 - 11

C: nach Abkühlung Zugeben von 150 ml Ethanol, Abfiltrieren des Niederschlages, Waschen mit Ethanol und Umkristallisieren aus Essigsäure

D: nach Abkühlung Zugeben von 65 ml Ethanol, Abfiltrieren des Niederschlages, Digerieren des Rohproduktes mit 310 ml heißem Dimethylformamid, Abfiltrieren des gereinigten Produktes und Waschen mit Ethanol

E: nach Abkühlung Zugeben von 85 ml Methanol, Abfiltrieren des Niederschlages, Waschen mit Methanol und Aceton, Lösen des Rohproduktes in einer heißen Mischung aus 85 ml Essigsäure und 125 ml Ameisensäure und Wiederausfällen durch Eingießen dieser Lösung in Wasser

F: Abfiltrieren des Niederschlages, Waschen mit wenig Methanol und Umkristallisieren aus Acetonitril

G: nach Abkühlung Abfiltrieren des Niederschlages und Waschen mit Ethanol

Anwendungsbeispiele

Stabilisierung von Polyamid: Beispiele 9 bis 14

Zur Herstellung von Belichtungsproben wurden jeweils 0,2 Gew.-Teile des Stabilisators aus den Beispielen 1 bis 5 bzw. eines Stabilisators des Standes der Technik in 100 Gew.-Teile Polyamid-6 durch einmaliges Extrudieren bei 250 °C eingearbeitet und das anfallende Granulat wurde über eine Spritzgießmaschine bei 250 °C zu 2 mm dicken Prüfkörpern gespritzt.

Diese Prüfkörper wurden in einem Xenotest® 1200-Schnellbewitterungsgerät der Firma Hanau auf ihre Licht- und Wetterechtheit getestet. Die Alterung wurde durch Messen der Zeit bis zum Beginn der Rißbildung an der Oberfläche der Prüfkörper bestimmt.

Die Ergebnisse sind in der unten aufgeführten Tabelle 2 zusammengestellt.

Tabelle 2

Stabilisierung von Polyamid-6

| Beispiel Nr. | Stabilisator aus Beispiel | Zeit bis zur Rißbildung |
|---|---|---|
| erfindungsgemäß: | | |
| 9 | 1 | > 2250 h |
| 10 | 2 | 2250 h |
| 11 | 3 | > 2250 h |
| 12 | 4 | > 2250 h |
| 13 | 5 | 2250 h |
| zum Vergleich: | | |
| 14 | * | 1750 h |

* Verbindung 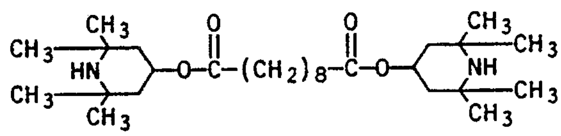

gemäß R. Gächter und H. Müller, Taschenbuch der Kunststoff-Additive, 2. Ausgabe, Carl Hanser Verlag München Wien 1983, S. 189

## Ansprüche

1. Cyclische Amide der allgemeinen Formel I

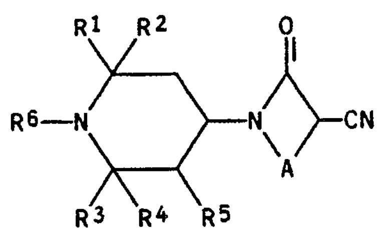

I

in der die Variablen folgende Bedeutung haben:
A eine Gruppierung der folgenden Formel

(a) 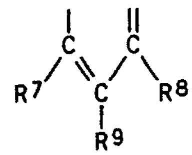

(b) 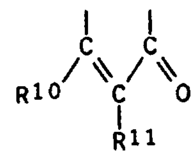

(c) 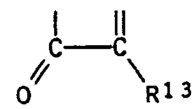

(d) 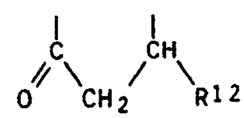

(e) 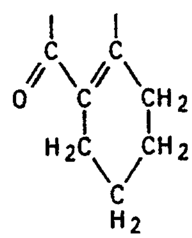

wobei jeweils die linke freie Bindung die Verknüpfung mit dem Amid-Stickstoff herstellt, die rechte freie Bindung je nach Gruppierung eine Einfach- oder eine Doppelbindung ist und wobei die Reste $R^7$ bis $R^{12}$ für Wasserstoff, $C_1$-$C_4$-Alkylgruppen, Phenylgruppen und der Rest $R^{13}$ für einen der Reste $R^7$ bis $R^{12}$ oder eine Hydroxylgruppe steht

$R^1$ bis $R^4$ $C_1$-$C_4$-Alkylgruppen, wobei $R^1$ und $R^2$ und/oder $R^3$ und $R^4$ jeweils zu einem fünf- oder sechsgliedrigen Ring verbunden sein können

$R^5$ Wasserstoff oder eine $C_1$-$C_3$-Alkylgruppe

$R^6$ Wasserstoff, eine $C_1$-$C_4$-Alkylgruppe, die zusätzlich durch eine Cyano-, Hydroxyl- oder Aminogruppe oder einen Phenylrest substituiert sein kann, oder eine $C_1$-$C_4$-Acylgruppe.

2. Cyclische Amide I nach Anspruch 1, bei denen die Variablen folgende Bedeutung haben:

$R^1$ bis $R^4$ Methylgruppen

$R^5$ und $R^6$ Wasserstoff

$R^7$, $R^8$ und $R^{10}$ Methyl- oder Ethylgruppen oder Phenylreste

$R^9$ und $R^{11}$ Wasserstoff oder Methylgruppen

$R^{12}$ eine Phenylgruppe

$R^{13}$ eine Hydroxylgruppe.

3. Verfahren zur Herstellung der cyclischen Amide I gemäß Anspruch 1 oder 2, dadurch gekennzeichnet, daß man α-Cyano-N-(polyalkylpiperidyl)acetamide der allgemeinen Formel II

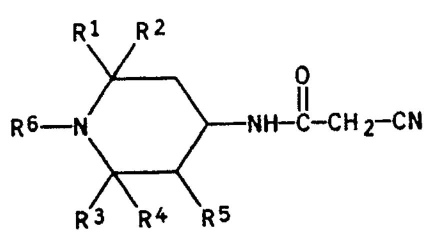 II

(a) mit einer 1,3-Dicarbonylverbindung,
(b) mit einem β-Carbonylcarbonsäureester,
(c) mit einem Oxalsäurediester oder α-Carbonylcarbonsäureester,
(d) mit einem olefinisch α,β-ungesättigten Carbonsäureester oder
(e) mit einem Cyclohexanon-2-carbonsäureester
umsetzt.

4. Verwendung von cyclischen Amiden I gemäß Anspruch 1 oder 2 als Stabilisatoren für organische Materialien.

5. Gegen die Einwirkung von Licht und Wärme stabilisierte organische Materialien, enthaltend 0,01 bis 5 Gew.-%, bezogen auf die Menge des organischen Materials, eines cyclischen Amids I gemäß Anspruch 1 oder 2.

6. Gegen die Einwirkung von Licht und Wärme stabilisierte Kunststoffe und Lacke, enthaltend 0,01 bis 5 Gew.-%, bezogen auf die Menge des Kunststoffes oder Lackes, eines cyclischen Amids I gemäß den Anspruch 1 oder 2.

Europäisches Patentamt

# EUROPÄISCHER RECHERCHENBERICHT

Nummer der Anmeldung

EP 90 11 8943

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| Y | EP-A-0 241 419 (SANDOZ)<br>* Seite 17, unten; Verbindung XXV; Beispiel 2 *<br>--- | 1-6 | C 07 D 401/04<br>C 07 D 211/88<br>C 08 K 5/3435 |
| Y | US-A-4 778 837 (WATERMAN)<br>* Insgesamt *<br>----- | 1-6 | |

| RECHERCHIERTE SACHGEBIETE (Int. Cl.5) |
|---|
| C 07 D 211/00<br>C 07 D 401/00 |

Der vorliegende Recherchenbericht wurde für alle Patentansprüche erstellt

| Recherchenort | Abschlußdatum der Recherche | Prüfer |
|---|---|---|
| DEN HAAG | 15-01-1991 | KISSLER B.E. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)